(19)

Europäisches
Patentamt
European
Patent Office
Office européen
des brevets

(11)  EP 3 698 704 A1

(12)  EUROPEAN PATENT APPLICATION

(43) Date of publication:
26.08.2020  Bulletin 2020/35

(21) Application number: 19158278.2

(22) Date of filing: 20.02.2019

(51) Int Cl.:
A61B 5/00 (2006.01)
A61B 5/024 (2006.01)
A61B 5/1455 (2006.01)
A61B 5/0205 (2006.01)
A61B 5/11 (2006.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Koninklijke Philips N.V.
5656 AG Eindhoven (NL)

(72) Inventor: DE HAAN, Gerard
5656 AE Eindhoven (NL)

(74) Representative: de Haan, Poul Erik et al
Philips International B.V.
Philips Intellectual Property & Standards
High Tech Campus 5
5656 AE Eindhoven (NL)

(54) DEVICE, SYSTEM AND METHOD FOR DETERMINING PHYSIOLOGICAL INFORMATION

(57)  The present invention relates to a device, system and method for determining physiological information indicative of at least one vital sign of a subject. The device comprises a pulse signal computation unit (132) configured to compute a pulse signal (211) from at least three detection signals (210), wherein at least one of the detection signals comprises wavelength-dependent reflection or transmission information in a wavelength channel below 600 nm. A processing unit (133) derives physiological information (212) indicative of at least one vital sign from a part of said signature vector represented by vector coefficients that reflect pulsatilities of the detection signals comprising wavelength-dependent reflection or transmission information in wavelength channels above 600 nm.

FIG.3

EP 3 698 704 A1

**Description**

FIELD OF THE INVENTION

The present invention relates to a device, system and method for determining physiological information indicative of at least one vital sign of a subject, such as a patient.

BACKGROUND OF THE INVENTION

Vital signs of a person, for example the heart rate (HR), the respiration rate (RR) or the arterial blood oxygen saturation ($SpO_2$), serve as indicators of the current state of a person and as powerful predictors of serious medical events. For this reason, vital signs are extensively monitored in inpatient and outpatient care settings, at home or in further health, leisure and fitness settings.

One way of measuring vital signs is plethysmography. Plethysmography generally refers to the measurement of volume changes of an organ or a body part and in particular to the detection of volume changes due to a cardio-vascular pulse wave traveling through the body of a subject with every heartbeat.

Photoplethysmography (PPG) is an optical measurement technique that evaluates a time-variant change of light reflectance or transmission of an area or volume of interest. PPG is based on the principle that blood absorbs light more than surrounding tissue, so variations in blood volume with every heart beat affect transmission or reflectance correspondingly. Besides information about the heart rate, a PPG waveform (also called PPG signal) can comprise information attributable to further physiological phenomena such as the respiration. By evaluating the transmittance and/or reflectivity at different wavelengths (typically red and infrared), the blood oxygen saturation can be determined.

Conventional pulse oximeters (also called contact PPG device herein) for measuring the heart rate and the (arterial) blood oxygen saturation of a subject are attached to the skin of the subject, for instance to a fingertip, earlobe or forehead. Therefore, they are referred to as 'contact' PPG devices. Although contact PPG is regarded as a basically non-invasive technique, contact PPG measurement is often experienced as being unpleasant and obtrusive, since the pulse oximeter is directly attached to the subject and any cables limit the freedom to move and might hinder a workflow.

Non-contact, remote PPG (rPPG) devices (also called camera-based device) for unobtrusive measurements have been proposed in the last decade. Remote PPG utilizes light sources or, in general radiation sources, disposed remotely from the subject of interest. Similarly, also a detector, e.g., a camera or a photo detector, can be disposed remotely from the subject of interest. Therefore, remote photoplethysmographic systems and devices are considered unobtrusive and well suited for medical as well as non-medical everyday applications.

Using PPG technology, vital signs can be measured, which are revealed by minute light absorption changes in the skin caused by the pulsating blood volume, i.e. by periodic color changes of the human skin induced by the blood volume pulse. As this signal is very small and hidden in much larger variations due to illumination changes and motion, there is a general interest in improving the fundamentally low signal-to-noise ratio (SNR). There still are demanding situations, with severe motion, challenging environmental illumination conditions, or high required accuracy of the application, where an improved robustness and accuracy of the vital sign measurement devices and methods is required, particularly for the more critical healthcare applications.

Video Health Monitoring (Heart Rate, respiration rate, SpO2, actigraphy, delirium, etc.) is a promising emerging field. Its inherent unobtrusiveness has distinct advantages for patients with fragile skin, or in need of long-term vital signs monitoring, such as NICU patients, patients with extensive burns, or COPD patients who have to be monitored at home during sleep. In other settings such as in a general ward or emergency department, the comfort of contactless monitoring is still an attractive feature.

While a promising new field, many challenges have to be overcome. Designing the system to be robust to movements of the patient is currently one of the main challenges, particularly to enable application in the emergency department.

SUMMARY OF THE INVENTION

It is an object of the present invention to provide a device, system and method for determining physiological information indicative of at least one vital sign of a subject by which results with higher reliability even in case of movements of the subject can be achieved.

In a first aspect of the present invention a device for determining physiological information indicative of at least one vital sign of a subject is presented, said device comprising:

-    an input interface configured to obtain at least three detection signals derived from detected electromagnetic radiation transmitted through or reflected from a skin region of a subject, wherein each detection signal comprises wavelength-dependent reflection or transmission information in a different wavelength channel, wherein at least one of the detection signals comprises wavelength-dependent reflection or transmission information in a wavelength channel below 600 nm,

- a pulse signal computation unit configured to compute a pulse signal from said at least three detection signals using a given signature vector representing expected relative pulsatilities of the pulse signal in the at least three detection signals, wherein the computation of the pulse signal involves computing a weighted combination of the at least three detection signals using weights resulting in a pulse signal for which the product with the obtained at least three detection signals equals the relative pulsatilities as represented by the signature vector,
- a processing unit for deriving physiological information indicative of at least one vital sign from a part of said signature vector represented by vector coefficients that reflect pulsatilities of the detection signals comprising wavelength-dependent reflection or transmission information in wavelength channels above 600 nm.

In a further aspect of the present invention a system for determining physiological information indicative of at least one vital sign of a subject is presented, said system comprising:

- a detector for detecting electromagnetic radiation transmitted through or reflected from a skin region of a subject and for deriving at least three detection signals from the detected electromagnetic radiation, wherein each detection signal comprises wavelength-dependent reflection or transmission information in a different wavelength channel, wherein one of the detection signal comprises wavelength-dependent reflection or transmission information in a wavelength channel below 600 nm,
- a device as disclosed herein for extracting physiological information.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The present invention is based on the idea to use the high-pulsatile wavelengths for motion-robust determination of physiological information, in particular measurements of vital signs such as $SpO_2$ (and others), to further improve motion robustness, without introducing the need for recalibration. A problem for $SpO_2$ methods is that for calibratability (i.e. the method can be calibrated to yield a good accuracy on different subjects, and is not too critical regarding available skin-regions) it is required to use wavelengths longer than 650 nm, since the much stronger PPG signal around 550 nm varies too much between individuals and skin sites. Hence, according to the present invention the highly pulsatile wavelength below 600 nm (e.g. around 550 nm) is used in a signature-based indirect $SpO_2$-estimation (or determination of other physiological information), but to neglect the pulsatility (the relative pulsatile strength, i.e. AC/DC of the reflected light) at this wavelength below 600 nm itself to maintain good calibratability.

As explained above, a PPG signal results from variations of the blood volume in the skin. Hence, the variations give a characteristic pulsatility "signature" when viewed in different spectral components of the reflected/transmitted light. This "signature" is basically resulting as the contrast (difference) of the absorption spectra of the blood and that of the blood-less skin tissue (wherein the scattering properties of the skin also plays a role). If the detector, e.g. a camera or sensor, has a discrete number of color channels, each sensing a particular part of the light spectrum, then the relative pulsatilities, i.e. the time-varying part divided by the average over time (AC/DC), in these channels can be arranged in a "signature vector", also referred to as the "normalized blood-volume vector", PBV. It has been shown in G. de Haan and A. van Leest, "Improved motion robustness of remote-PPG by using the blood volume pulse signature", Physiol. Meas. 35 1913, 2014, which is herein incorporated by reference, that if this signature vector is known, then a motion-robust pulse signal extraction on the basis of the color channels and the signature vector is possible. For the quality of the pulse signal it is essential though that the signature is accurate, as otherwise the known methods mixes noise into the output pulse signal in order to achieve the prescribed correlation of the pulse vector with the normalized color channels as indicated by the signature vector.

Details of the PBV method and the use of the normalized blood volume vector (called "predetermined index element having a set orientation indicative of a reference physiological information") have also been described in US 2013/271591 A1, which details are also herein incorporated by reference.

The present invention thus achieves motion robust $SpO_2$ measurement using a vital signs camera, but also results in an improved motion robust pulse signal, an improved pulse rate, optionally an improved serum bilirubin estimation (essentially all PPG-based information can be made more robust). Finally, the invention may even be used to improve motion robustness of contact oximeters.

In a preferred embodiment two of said at least three detection signals comprise wavelength-dependent reflection or transmission information in different wavelength channels of red and/or infrared light and the third detection signal comprises wavelength-dependent reflection or transmission information in a wavelength channel of visible light of another color than red. In a particular implementation a first wavelength channel covers a wavelength or wavelength range in the range of 620 nm to 1000 nm, in particular in the range of 660 to 760 nm, a second wavelength channel covers a wavelength or wavelength range in the range of 620 nm to 1000 nm, in particular in the range of 850 to 940 nm (e.g. around 900 nm), and a third wavelength channel covers a wavelength or wavelength range in the range of 400 nm to 600 nm, in particular in the range of 520 to 580 nm. Using these wavelength channels has shown to provide reliable results, particular regarding the determination of one or more vital signs such as SpO2.

The processing unit may further be configured to derive physiological information indicative of the relative concentration of a blood species, in particular of one or more of Hb, $HbO_2$, MetHb, and HbCO. The invention has shown to be particularly useful in determining such physiological information.

In a further embodiment said pulse signal computation unit is configured to compute at least two pulse signals from said at least three detection signals using different given signature vectors, wherein for the computation of each pulse signal a different given signature vector is used, wherein a given signature vector represents expected relative pulsatilities of the respective pulse signal in the at least three detection signals, wherein the computation of a pulse signal involves computing a weighted combination of the at least three detection signals using weights resulting in a pulse signal for which the product with the obtained at least three detection signals equals the relative pulsatilities as represented by the respective signature vector, wherein said device further comprises a quality indicator computation unit for computing quality indicator values for said pulse signals indicating a characteristic of the respective pulse signal, and wherein said processing unit is configured to derive physiological information indicative of at least one vital sign using a part of one or more signature vectors that result in the one or more pulse signals with the best quality indicator values and that is represented by vector coefficients that reflect pulsatilities of the detection signals comprising wavelength-dependent reflection or transmission information in wavelength channels above 600 nm (i.e. in channels with wavelengths longer than 600 nm).

The characteristic wavelength-dependency of the PPG signal varies when the composition of the blood changes. Particularly, oxygen-saturation of arterial blood has a strong effect on the absorption in the wavelength range between 620nm and 780nm. This changing signature for different $SpO_2$ values leads to relative PPG pulsatility that depends on the arterial blood oxygenation. This dependency can be used to realize a motion-robust remote $SpO_2$ monitoring system that has been named adaptive PBV method (APBV) and is described in detail in WO 2017/055218 A1 and in M. van Gastel, S. Stuijk and G. de Haan, "New principle for measuring arterial blood oxygenation, enabling motion-robust remote monitoring", Nature Scientific Reports, Nov. 2016. The description of the details of the APBV method provided in these documents is also herein incorporated by reference.

This embodiment may be further refined when said pulse signal computation unit is configured to use a fixed set of different signature vectors and said processing unit is configured to filter a time sequence of said part of the signature vectors that resulted in the pulse signal with the best quality indicator value to obtain a filtered partial signature vector from which the physiological information is derived, wherein said time sequence of partial signature vectors is obtained from pulse signals and quality indicators computed for successive time windows of said at least two detection signals.

There are various options for determining and using the quality indicator. In one embodiment the quality indicator computation unit may be configured to compute the magnitude spectrum of normalized pulse signals (e.g. pulse signal divided by its standard deviation), in particular over a sliding time window, and to use an amplitude of the highest peak in a range, in particular of typical pulse frequencies, as the quality indicator for said pulse signal. Rather than using the magnitude spectrum of the normalized pulse signal as a basis for the computation, the order may be reversed, i.e. the normalized magnitude spectrum of the pulse signal may be use, where normalization may be a division of the magnitude spectrum by its sum or by its energy.

In another embodiment the quality indicator computation unit may be configured to compute pulse signals in a sliding time window and to define the skewness in the range of the pulse frequencies of the spectrum, as the quality indicator for said pulse signal.

In yet another embodiment the quality indicator computation unit may be configured to compute band-pass filtered (limited to HR-band) pulse signals in a sliding time window and to define the kurtosis of a pulse signal as the quality indicator for said pulse signal.

In still another embodiment the pulse signal computation unit may be configured to compute said pulse signals $S_1$, $S_2$ by computing a covariance matrix $Q = C_n C_n^T$ of normalized DC-free detection signals $C_n$ over a time window and find the weights $W_x$ to compute a pulse signal $S_x = \overrightarrow{W_x} C_n$ as $\overrightarrow{W_x} = k\overrightarrow{P_{bvx}}Q^{-1}$, where k is chosen to make $\|\overrightarrow{W_x}\| = 1$ and x $\in \{1, 2\}$. It shall be noted here that the weights and the $P_{bv}$s are different for the two pulse signals obtained from the

same detection signals $C_n$.

BRIEF DESCRIPTION OF THE DRAWINGS

These and other aspects of the invention will be apparent from and elucidated with reference to the embodiment(s) described hereinafter. In the following drawings

Fig. 1 shows a diagram of the absorption spectrum of blood;
Fig. 2 shows a schematic diagram of a system according to the present invention;
Fig. 3 shows a schematic diagram of a first embodiment of a device according to the present invention;
Fig. 4 shows a schematic diagram of a second embodiment of a device according to the present invention; and
Fig. 5 shows a diagram of an ordered signature vector set.

DETAILED DESCRIPTION OF THE INVENTION

A problem with known methods for determining physiological information indicative of at least one vital sign of a subject is their sensitivity for subject motion. The reason for this is that the vital signs manifest themselves as small color variations of the skin that are detected by a non-contact sensor (e.g. a camera) or a contact sensor (e.g. a pulse oximeter). Subject motion, however, leads easily to much larger variations in the detected signals, and recovering the vital sign from these distorted signals is one of the main issues to be solved. The above described PPV and APPV methods solve this problem by using a signature-based pulse extraction method to indirectly measure e.g. $SpO_2$ in a robust fashion. Even though this resulted in a huge step in robustness, it may still be further improved to allow application e.g. in the emergency department where motions are typically stronger than for a patient in bed.

A problem for $SpO_2$ estimation methods is that for calibratability it is required to use wavelengths longer than 650 nm. This is because shorter wavelengths penetrate the skin poorly. Consequently, the pulsatility becomes strongly dependent on thickness of skin layers, which varies amongst subjects and body sites. The low pulsatility at longer wavelengths, however, increases the vulnerability for subject motion. It would be very attractive to use the much (approximately 5-10 times) stronger PPG signal around 550nm, as this could improve motion robustness with the same factor.

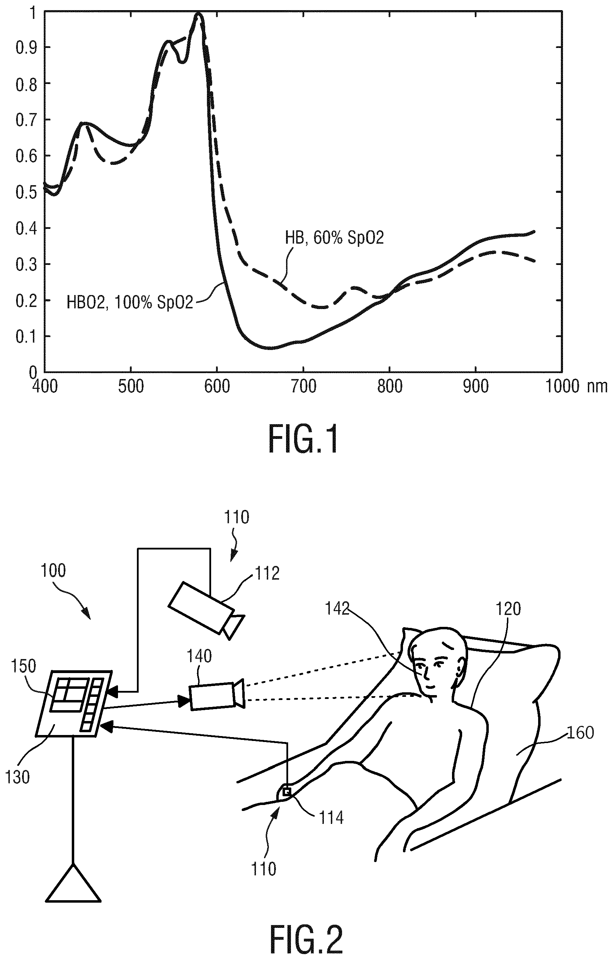

This can be seen in Fig. 1 showing a diagram of the absorption spectrum of blood which depends on the oxygen saturation. If a sensor samples this spectrum, e.g. around 650/760 nm, 810 nm and 900 nm, the "signature" vector, Pbv, has three components that all depend on the $SpO_2$.

Also, unlike sleep-monitoring applications, measurements in the emergency department may use visible light without being considered obtrusive. Unfortunately, experiments show that this much higher pulsatility varies rather much between individuals and skin sites and depend on environmental conditions, which would require a recalibration for each subject and possibly even repetition of this procedure with environmental changes (e.g. in temperature).

The present invention uses the high-pulsatile wavelengths for motion-robust determination of physiological information, in particular for determining vital signs such as $SpO_2$. One element of the present invention is to use, in addition to or as an alternative of one of the NIR wavelengths used in known systems, a wavelength below 600 nm, e.g. around 550 nm, where the pulsatility is very high, but in a relative broadly varying range insignificant to the determination of the physiological information, in particular the determination of the $SpO_2$ value. In this way motion robustness can be improved without introducing the need for recalibration. This will be explained in more detail below.

Fig. 2 shows a schematic diagram of an embodiment of a system 100 according to the present invention. The system 100 comprises a detector 110 for detecting electromagnetic radiation transmitted through or reflected from a skin region of a subject 120 and for deriving at least three detection signals from the detected electromagnetic radiation. Each detection signal comprises wavelength-dependent reflection or transmission information in a different wavelength channel, wherein one of the detection signal comprises wavelength-dependent reflection or transmission information in a wavelength channel below 600 nm (i.e. in a channel with wavelengths shorter than 600 nm).

The system 100 further comprises a device 130 for determining physiological information indicative of at least one vital sign of a subject from the at least three detection signals. The subject 120 maybe a patient, in this example a patient lying in a bed 160, e.g. in a hospital or other healthcare facility, but may also be a neonate or premature infant, e.g. lying in an incubator, or a person at home or in a different environment.

There exist different embodiments for a detector for detecting electromagnetic radiation transmitted through or reflected from a subject, which may alternatively (which is preferred) or together be used. In the embodiment of the system 100, two different embodiments of the detector are shown and will be explained below. Both embodiments of the detector are configured for deriving detection signals from the detected electromagnetic radiation, wherein each detection signal comprises wavelength-dependent reflection or transmission information in a different wavelength channel. Hereby, optical filters may be used which are preferably different, though their filter bandwidth can be overlapping.

It is sufficient if their wavelength-dependent transmission is different.

In one embodiment the detector comprises a camera 112 (also referred to as imaging unit, or as camera-based or remote PPG sensor) including a suitable photosensor for (remotely and unobtrusively) capturing image frames of the subject 120, in particular for acquiring a sequence of image frames of the subject 120 over time, from which photoplethysmography signals can be derived. The image frames captured by the camera 112 may particularly correspond to a video sequence captured by means of an analog or digital photosensor, e.g. in a (digital) camera. Such a camera 112 usually includes a photosensor, such as a CMOS or CCD sensor, which may also operate in a specific spectral range (visible, IR) or provide information for different spectral ranges. The camera 112 may provide an analog or digital signal. The image frames include a plurality of image pixels having associated pixel values. Particularly, the image frames include pixels representing light intensity values captured with different photosensitive elements of a photosensor. These photosensitive elements may be sensitive in a specific spectral range (i.e. representing a specific color or pseudo-color (in NIR)). The image frames include at least some image pixels being representative of a skin portion of the subject. Thereby, an image pixel may correspond to one photosensitive element of a photo-detector and its (analog or digital) output or may be determined based on a combination (e.g. through binning) of a plurality of the photosensitive elements.

In another embodiment the detector comprises one or more optical photoplethysmography sensor(s) 114 (also referred to as contact PPG sensor(s)) configured for being mounted to a skin portion of the subject 120 for acquiring photoplethysmography signals. The PPG sensor(s) 114 may e.g. be designed in the form of a finger-clip or as a wrist-worn wearable device for measuring the blood oxygen saturation or a heart rate sensor for measuring the heart rate, just to name a few of all the possible embodiments.

When using a camera 112 the system 100 may further optionally comprise a light source 140 (also called illumination source), such as a lamp, for illuminating a region of interest 142, such as the skin of the patient's face (e.g. part of the cheek or forehead), with light, for instance in a predetermined wavelength range or ranges (e.g. in the red, green and/or infrared wavelength range(s)). The light reflected from said region of interest 142 in response to said illumination is detected by the camera 112. In another embodiment no dedicated light source is provided, but ambient light is used for illumination of the subject 120. From the reflected light only light in a number of desired wavelength ranges (e.g. green and red or infrared light, or light in a sufficiently large wavelength range covering at least two wavelength channels) may be detected and/or evaluated.

The device 130 is further connected to an interface 150 for displaying the determined information and/or for providing medical personnel with an interface to change settings of the device 130, the camera 112, the PPG-sensor(s) 114, the light source 140 and/or any other parameter of the system 100. Such an interface 150 may comprise different displays, buttons, touchscreens, keyboards or other human machine interface means.

A system 100 as illustrated in Fig. 2 may, e.g., be located in a hospital, healthcare facility, elderly care facility or the like. Apart from the monitoring of patients, the present invention may also be applied in other fields such as neonate monitoring, general surveillance applications, security monitoring or so-called live style environments, such as fitness equipment, a wearable, a handheld device like a smartphone, or the like. The uni- or bidirectional communication between the device 130, the camera 112, the PPG sensor(s) 114 and the interface 150 may work via a wireless or wired communication interface. Other embodiments of the present invention may include a device 130, which is not provided stand-alone, but integrated into the camera 112 or the interface 150.

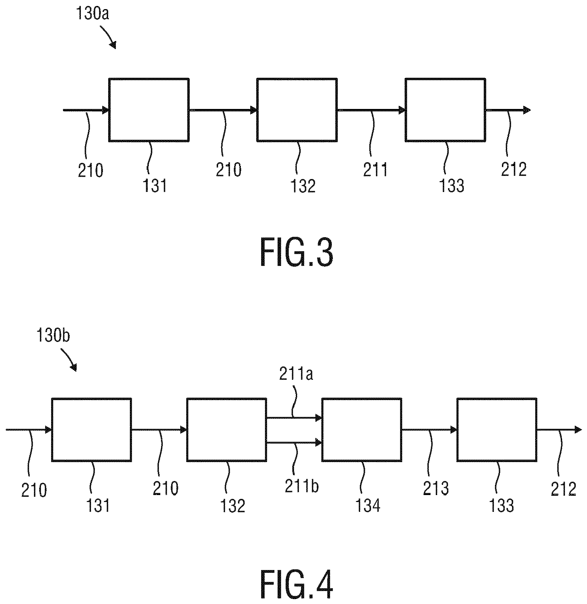

Fig. 3 shows a more detailed schematic illustration of a first embodiment 130a of the device 130 according to the present invention. The device 130a comprises an input interface 131 configured to obtain at least three detection signals 210 derived from detected electromagnetic radiation transmitted through or reflected from a skin region of the subject 120. As mentioned above, each detection signal 210 comprises wavelength-dependent reflection or transmission information in a different wavelength channel, wherein at least one of the detection signals 210 comprises wavelength-dependent reflection or transmission information in a wavelength channel below 600 nm.

The device 130a further comprises a pulse signal computation unit 132 configured to compute a pulse signal 211 from said at least three detection signals 211 using a given signature vector representing expected relative pulsatilities of the pulse signal in the at least three detection signals 210. The computation of the pulse signal 211 involves computing a weighted combination of the at least three detection signals 210 using weights resulting in a pulse signal for which the (inner) product with the obtained (original) at least three detection signals equals the relative pulsatilities as represented by the signature vector. Preferable, the pulse signal and the detection signals are made DC-free and normalized before performing this computation.

The device 130a further comprises a processing unit 133 for deriving physiological information 212 indicative of at least one vital sign from a part of said signature vector represented by vector coefficients that reflect pulsatilities of the detection signals comprising wavelength-dependent reflection or transmission information in wavelength channels above 600 nm.

The various units of the device 130a may be comprised in one or multiple digital or analog processors depending on how and where the invention is applied. The different units may completely or partly be implemented in software and carried out on a personal computer connected to one or more detectors. Some or all of the required functionality may also be implemented in hardware, e.g. in an application specific integrated circuit (ASIC) or in a field programmable gate array (FPGA).

Hence, according to this embodiment of the present invention it is ensured that a highly pulsatile wavelength below 600 nm, e.g. around 550 nm in a (preferably signature-based, i.e. indirect), is used in the determination of the physiological information, e.g. in an $SpO_2$ estimation, in addition to at least two wavelengths above 600 nm (preferably above 630 nm), whereby it is made sure that the pulsatile strength of the wavelength below 600 nm itself plays no role in the resulting physiological information (e.g. $SpO_2$) to maintain good calibratability.

The relative pulsatilities at the wavelengths used can be described as a characteristic signature, i.e. a vector with components reflecting the relative pulsatile strength at the wavelengths used. One of these components corresponds to the pulsatile strength of the wavelength below 600 nm, e.g. at around 550 nm, which is fundamentally uncertain and carries no information.

This "signature" has also been called "normalized blood-volume vector" PBV as explained above. If this PBV is known then a motion-robust pulse signal extraction on the basis of the color channels and the signature PBV is possible. For the quality of the pulse signal it is essential though that the signature is accurate as otherwise the above described PBV and APBV methods mixes noise into the output pulse signal in order to achieve the prescribed correlation of the pulse vector with the normalized color channels as indicated by the signature PBV. Testing multiple signature vectors in parallel, or adapting the signature using a quality metric, it is indirectly possible to know the composition of the blood ($SpO_2$, CO, MtHB, bilirubin, etc.), as the signature with the correct relative pulsatilities will give the highest pulse quality using the PBV method.

Fig. 4 shows a more detailed schematic illustration of a first embodiment 130b of the device 130 according to the present invention. According to this embodiment 130b the pulse signal computation unit 132 computes not just one, but at least two pulse signals 211a, 211b from said at least three detection signals 210 using different given signature vectors. For the computation of each pulse signal a different given signature vector is used, wherein a given signature vector represents expected relative pulsatilities of the respective pulse signal in the at least three detection signals, wherein the computation of a pulse signal involves computing a weighted combination of the at least three detection signals using weights resulting in a pulse signal for which the products with the obtained (original) at least three detection signals equals the relative pulsatilities as represented by the respective signature vector.

The device further comprises a quality indicator computation unit 134 for computing quality indicator values 213 for said pulse signals 211a, 211b indicating a characteristic of the respective pulse signal. The processing unit 133 is configured to derive physiological information 212 indicative of at least one vital sign from a part of the (one or more) signature vector(s) that result(s) in the pulse signal(s) with the best quality indicator value(s) 213 and that is (are) represented by vector coefficients that reflect pulsatilities of the detection signals comprising wavelength-dependent reflection or transmission information in wavelength channels above 600 nm. Hence, one only a part of a single signature vector (resulting in the best quality indicator value) may be used, but also parts of several (e.g. three) signature vectors (resulting in the best quality indicator values) may be used.

Thus, according to the present invention, contrary to the known described PBV and APBV methods, the additional wavelength channel below 600 nm (e.g. around 550 nm) requires an extension of the range of vectors to be tested, although the choice of the component that corresponds to the pulsatile strength of this wavelength is disregarded in the determination of the physiological information, e.g. in the measurement of $SpO_2$, CO, MetHB, bilirubin, etc.

In another embodiment, a robust pulse signal and SpO2 measurement can be implemented using the APBV method with a sensor/camera e.g. sampling the spectrum at 660 nm, 880 nm, and additionally around 550 nm. The signature vector set, tested in parallel, using the PBV method, exhibits vectors with three components, reflecting the relative pulsatilities in the 550 nm, 660 nm and 880 nm channel, respectively.

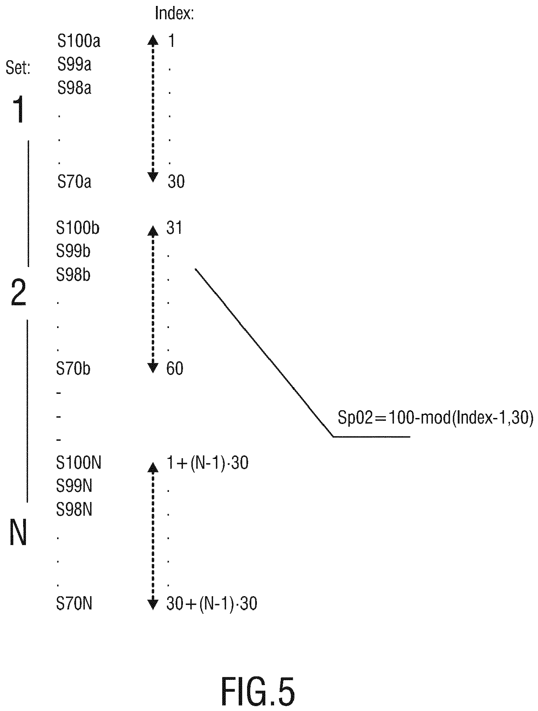

In a particular example, this set may include M=30 different combinations of 660 nm and 880 nm components, reflecting M=30 different $SpO_2$ values (e.g. 100% - 70% SpO2, in steps of 1%), which occur multiple (N) times with different first components (reflecting the uncertain relative 550 nm pulsatility). If these vectors are ordered in a list, as shown in Fig. 5, the index in this list corresponds to a particular $SpO_2$ value. Since the 550 nm pulsatility is to be neglected, the index may be taken modulo M to provide the $SpO_2$. Although the choice of subset is meaningless for the $SpO_2$ measurement, it may be used for other measurements (e.g. skin property, body site, pigmentation, etc.). As shown in Fig. 5, the ordered signature vector set contains repeating subsets with the relative pulsatilities in the red/infrared channels corresponding to the $SpO_2$ range, while each subset has a different pulsatility of the 550 nm channel. The index in the ordered list (signature with best quality pulse signal) identifies the $SpO_2$ value, while the modulo-function used prohibits any effect from the 550 nm pulsatile strength.

The table below shows a much simplified set of a suitable PBV signature vector set.

| SpO2 | 550nm | 660nm | 880nm |
|---|---|---|---|
| 100% | 7.0000 | 0.2000 | 1.0000 |
| | 7.0000 | 0.4500 | 1.0000 |
| 85% | 7.0000 | 0.7000 | 1.0000 |
| | 7.0000 | 0.9500 | 1.0000 |
| 70% | 7.0000 | 1.2000 | 1.0000 |
| | 7.0000 | 1.4500 | 1.0000 |
| 100% | 8.0000 | 0.2000 | 1.0000 |
| | 8.0000 | 0.4500 | 1.0000 |
| 85% | 8.0000 | 0.7000 | 1.0000 |
| | 8.0000 | 0.9500 | 1.0000 |
| 70% | 8.0000 | 1.2000 | 1.0000 |
| | 8.0000 | 1.4500 | 1.0000 |
| 100% | 9.0000 | 0.2000 | 1.0000 |
| | 9.0000 | 0.4500 | 1.0000 |
| 85% | 9.0000 | 0.7000 | 1.0000 |
| | 9.0000 | 0.9500 | 1.0000 |
| 70% | 9.0000 | 1.2000 | 1.0000 |
| | 9.0000 | 1.4500 | 1.0000 |

The second set of numbers (lines 7-12) show the relative pulsatilities for 550 nm, 660 nm and 880 nm as a function of $SpO_2$. The first set of numbers (lines 1-6) and the third set of numbers (lines 13-18) are copies thereof, where the pulsatility in the 550 nm channel has been decreased and increased, respectively. For the actual $SpO_2$ measurement it is irrelevant whether the optimal vector is found in the first, second or third vector subset, but only the relative position in the subset is relevant.

In the following some basic considerations with respect to the PBV method shall be briefly explained.

The beating of the heart causes pressure variations in the arteries as the heart pumps blood against the resistance of the vascular bed. Since the arteries are elastic, their diameter changes in sync with the pressure variations. These diameter changes occur even in the smaller vessels of the skin, where the blood volume variations cause a changing absorption of the light.

The unit length normalized blood volume pulse vector (also called signature vector) is defined as PBV, providing the relative PPG-strength in the red, green and blue camera signal, i.e.

$$\vec{P}_{bv} = \frac{\left[\sigma(\vec{R}_{n}), \sigma(\vec{G}_{n}), \sigma(\vec{B}_{n})\right]}{\sqrt{\sigma^2(\vec{R}_{n}) + \sigma^2(\vec{G}_{n}) + \sigma^2(\vec{B}_{n})}}$$

with $\sigma$ indicating the standard deviation.

To quantify the expectations, the responses Hred(w), $H_{green}$(w) and $H_{blue}$(w) of the red, green and blue channel, respectively, were measured as a function of the wavelength w, of a global-shutter color CCD camera1, the skin reflectance of a subject, $\rho_s$(w), and used an absolute PPG-amplitude curve PPG(w). From these curves, shown e.g. in Fig. 2 of the above cited paper of de Haan and van Leest, the blood volume pulse vector PBV is computed as:

$$\vec{\hat{P}}_{bv}^{T} = \begin{bmatrix} \dfrac{\int\limits_{w=400}^{700} H_{red}(w)I(w)PPG(w)\,dw}{\int\limits_{w=400}^{700} H_{red}(w)I(w)\rho_{s}(w)\,dw} \\ \dfrac{\int\limits_{w=400}^{700} H_{green}(w)I(w)PPG(w)\,dw}{\int\limits_{w=400}^{700} H_{green}(w)I(w)\rho_{s}(w)\,dw} \\ \dfrac{\int\limits_{w=400}^{700} H_{blue}(w)I(w)PPG(w)\,dw}{\int\limits_{w=400}^{700} H_{blue}(w)I(w)\rho_{s}(w)\,dw} \end{bmatrix}$$

which, using a white, halogen illumination spectrum I(w), leads to a normalized PBV = [0.27, 0.80, 0.54]. When using a more noisy curve the result maybe PBV = [0.29, 0.81, 0.50].

The blood volume pulse predicted by the used model corresponds reasonably well to an experimentally measured normalized blood volume pulse vector, PBV = [0.33, 0.78, 0.53] found after averaging measurements on a number of subjects under white illumination conditions. Given this result, it was concluded that the observed PPG-amplitude, particularly in the red, and to a smaller extent in the blue camera channel, can be largely explained by the crosstalk from wavelengths in the interval between 500 and 600 nm. The precise blood volume pulse vector depends on the color filters of the camera, the spectrum of the light and the skin-reflectance, as the model shows. In practice the vector turns out to be remarkably stable though given a set of wavelength channels (the vector will be different in the infrared compared to RGB-based vector).

It has further been found that the relative reflectance of the skin, in the red, green and blue channel under white illumination does not depend much on the skin-type. This is likely because the absorption spectra of the blood-free skin is dominated by the melanin absorption. Although a higher melanin concentration can increase the absolute absorption considerably, the relative absorption in the different wavelengths remains the same. This implies an increase of melanin darkens the skin, but hardly changes the normalized color of the skin. Consequently, also the normalized blood volume pulse PBV is quite stable under white illumination. In the infrared wavelengths the influence of melanin is further reduced as its maximum absorption occurs for short wavelengths (UV-light) and decreases for longer wavelengths.

The stable character of PBV can be used to distinguish color variations caused by blood volume change from variations due to alternative causes, i.e. the stable PBV can be used as a "signature" of blood volume change to distinguish their color variations. The known relative pulsatilities of the color channels PBV can thus be used to discriminate between the pulse-signal and distortions. The resulting pulse signal S using known methods can be written as a linear combination (representing one of several possible ways of "mixing") of the individual DC-free normalized color channels:

$$S = W\,C_{n}$$

with $WW^{T} = 1$ and where each of the three rows of the 3 x N matrix $C_{n}$ contains N samples of the DC-free normalized red, green and blue channel signals $R_{n}$, $G_{n}$ and $B_{n}$, respectively, i.e.:

$$\vec{R}_{n} = \frac{1}{\mu(\vec{R})}\vec{R} - 1, \quad \vec{G}_{n} = \frac{1}{\mu(\vec{G})}\vec{G} - 1, \quad \vec{B}_{n} = \frac{1}{\mu(\vec{B})}\vec{B} - 1.$$

Here the operator $\mu$ corresponds to the mean. Key difference between the different methods is in the calculation of the weighting vector W. In one method, the noise and the PPG signal may be separated into two independent signals built as a linear combination of two color channels. One combination approximated a clean PPG signal, the other contained noise due to motion. As an optimization criterion the energy in the pulse signal may be minimized. In another method a linear combination of the three color channels may be used to obtain the pulse signal.

The PBV method generally obtains the mixing coefficients using the blood volume pulse vector as basically described in US 2013/271591 A1 and the above cited paper of de Haan and van Leest. The best results are obtained if the band-passed filtered versions of $R_{n}$, $G_{n}$ and $B_{n}$ are used. According to this method the known direction of PBV is used to discriminate between the pulse signal and distortions. This not only removes the assumption (of earlier methods) that the pulse is the only periodic component in the video, but also eliminates assumptions on the orientation of the distortion signals. To this end, it is assumed as before that the pulse signal is built as a linear combination of normalized color signals. Since it is known that the relative amplitude of the pulse signal in the red, green and blue channel is given by PBV, the weights, $W_{PBV}$, are searched that give a pulse signal S, for which the correlation with the color channels $R_n$, $G_n$, and $B_n$ equals $P_{bv}$

$$\vec{S}C_n^T = k\vec{P}_{bv} \Leftrightarrow \vec{W}_{PBV}C_nC_n^T = k\vec{P}_{bv},\qquad(1)$$

and consequently the weights determining the mixing are determined by

$$\vec{W}_{PBV} = k\vec{P}_{bv}Q^{-1} \text{ with } Q = C_nC_n^T,\qquad(2)$$

and the scalar k is determined such that $W_{PBV}$ has unit length. It is concluded that the characteristic wavelength dependency of the PPG signal, as reflected in the normalized blood volume pulse, PBV, can be used to estimate the pulse signal from the time-sequential RGB pixel data averaged over the skin area. This algorithm is referred to as the PBV method.

According to an embodiment the reliability indicator may output a reliability value (regarding the quality of a pulse, or respiratory signal) depending on the similarity of the candidate pulses, according to a metric (e.g. same peak (pulse-rate) in frequency domain, or similar SNR, or low mean squared difference between pairs of pulse-signals).

In another embodiment the APBV method is used to extract an SpO2 value from two or more different combinations of three wavelength channels, e.g. from [λ1, λ2], from [λ1, λ3], and/or from [λ1, λ2, λ3]. In this case, the reliability indicator may output a reliability value depending on the similarity of the SpO2 values, according to a metric (e.g. standard deviation of candidate values (represented in Fig. 7), possibly filtered over a time-interval, and/or the SNR-values of the pulse-signals extracted by the different version of the APBV method, and/or (in case of a contact sensor) the similarity of the candidate SpO2 values in combination with the strength of a motion signal, e.g. an accelerometer signal, representative for patient motion, and/or (in case of a camera-based sensor) the strength of subject motion as represented by motion-vectors computed from the subject-video).

In the following some basic considerations with respect to the APBV method shall be briefly explained.

Instead of extracting features from the PPG waveforms, APBV determines SpO2 indirectly based on the signal quality of the pulse signals extracted with SpO2 'signatures'. This procedure can mathematically be described as:

$$SpO_2 = \underset{SpO_2 \in \mathbf{SpO_2}}{\mathrm{argmax}}\ SNR\left(\overbrace{k\vec{P}_{bv}(SpO_2)[C_nC_n^T]^{-1}C_n}^{\vec{W}_{PBV}}\right),\qquad(3)$$

where $C_n$ contains the DC-normalized color variations and scalar k is chosen such that $\vec{W}_{PBV}$ has unit length. The SpO2 signatures compiled in $\vec{P}_{bv}$ can be derived from physiology and optics. Assuming identical cameras the PPG amplitudes of N cameras can be determined by:

$$\vec{P_{bv}} = \left\| \begin{bmatrix} (\frac{AC}{DC})^1 \\ (\frac{AC}{DC})^2 \\ \vdots \\ (\frac{AC}{DC})^N \end{bmatrix} \right\| = \left\| \begin{bmatrix} \frac{\int_\lambda I(\lambda)F^1(\lambda)C(\lambda)PPG(\lambda)d\lambda}{\int_\lambda I(\lambda)F^1(\lambda)C(\lambda)\rho_s(\lambda)d\lambda} \\ \frac{\int_\lambda I(\lambda)F^2(\lambda)C(\lambda)PPG(\lambda)d\lambda}{\int_\lambda I(\lambda)F^2(\lambda)C(\lambda)\rho_s(\lambda)d\lambda} \\ \vdots \\ \frac{\int_\lambda I(\lambda)F^N(\lambda)C(\lambda)PPG(\lambda)d\lambda}{\int_\lambda I(\lambda)F^N(\lambda)C(\lambda)\rho_s(\lambda)d\lambda} \end{bmatrix} \right\|.$$

$$(4)$$

Here the PPG amplitude spectrum, PPG($\lambda$) can be approximated by a linear mixture of the light absorption spectra from the two most common variants of the main chromophore in arterial blood, hemoglobin; oxygenated (HbO2) and reduced (Hb):

$$PPG(\lambda) \approx \epsilon_{Hb}(\lambda)c_{Hb} + \epsilon_{HbO_2}(\lambda)c_{HbO_2} = (1 - SaO_2)\epsilon_{Hb}(\lambda) + SaO_2\epsilon_{HbO_2}(\lambda)$$
$$= \epsilon_{Hb}(\lambda) + SaO_2[\epsilon_{HbO_2}(\lambda) - \epsilon_{Hb}(\lambda)],$$

$$(5)$$

where it is assumed that the optical path length differences are negligible for 600 < $\lambda$ < 1000nm and SaO2 $\in$ [0, 1]. It is recognized that the wavelength-dependent effect of scattering could render this assumption invalid. When using two wavelengths the ratio-of-ratios parameter R and the ratio of APBV parameter $\vec{P_{bv}}$ coincide. The wavelength selection may be based on three criteria: 1) the desire to measure oxygen saturation in darkness ($\lambda$ > 700nm) for clinical applications, 2) have a reasonable SpO2 contrast, and 3) wavelengths within the spectral sensitivity of the camera. The idea to use three instead of the common two wavelengths used in pulse-oximetry was motivated by the improved robustness of the SpO2 measurement by a factor of two. This can be explained by how motion affects the PPG waveforms when measured with a camera. Since motion-induced intensity variations are equal for all wavelengths, suppression of these artifacts is possible for the APBV method if the pulse signature $\vec{P_{bv}}$ is not equal to this motion signature, which can be described as a vector with equal weights.

It shall be noted that even if the pulse quality is very good, it does not always mean that the estimated $SpO_2$ value is sufficiently reliable and can be trusted. This may particularly happen when unexpected blood-species (e.g. COHb) are available causing the $SpO_2$ calibration curve to shift, i.e. causing a different signature vector to lead to the optimal pulse quality when using the PBV method or APBV method for pulse extraction.

Although the indirect measurement of the relative pulsatilities, e.g. using APBV, seems most advantageous, alternative algorithms may be applied that use an additional, though less informative (e.g. 550 nm) wavelength channel to improve robustness of a measurement of a blood species concentration.

Typical embodiments use optical sensor channels conveying sensed reflection data in a wavelength range between 620 nm and 1000 nm, particularly 660/760nm and 880 nm, while the additional channel preferably conveys sensed reflection data in a range between 400 nm and 600 nm, particularly between 520 nm and 580 nm.

Typical applications of the present invention are in contact-sensor or camera-based photo-plethysmography where the primary measurement concerns the (relative) concentration of a blood species, e.g. Hb, $HbO_2$, MetHb, HbCO, bilirubin, etc., in a blood-vessel, particularly an artery or arteriole.

The present invention can be applied on detection signals that have been acquired using contact sensors and/or contactless sensors. By way of example, the present invention can be applied in the field of healthcare, e.g. unobtrusive remote patient monitoring, general surveillances, security monitoring and so-called lifestyle environments, such as fitness equipment, fitness/health-watches/bands (typically wrist-worn), or the like. Applications may include monitoring of oxygen saturation (pulse oximetry), heart rate, blood pressure, cardiac output, changes of blood perfusion, assessment of autonomic functions, respiration and detection of peripheral vascular diseases. The present invention can e.g. be used for rapid and reliable pulse detection of a critical patient, for instance during automated CPR (cardiopulmonary resuscitation). The system can be used for monitoring of vital signs of neonates with very sensitive skin e.g. in NICUs and for patients with damaged (e.g. burnt) skin, but may also be more convenient than contact sensors as used in the general ward.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device for determining physiological information indicative of at least one vital sign of a subject, said device comprising:

   - an input interface (131) configured to obtain at least three detection signals (210) derived from detected electromagnetic radiation transmitted through or reflected from a skin region of a subject, wherein each detection signal comprises wavelength-dependent reflection or transmission information in a different wavelength channel, wherein at least one of the detection signals comprises wavelength-dependent reflection or transmission information in a wavelength channel below 600 nm,
   - a pulse signal computation unit (132) configured to compute a pulse signal (211) from said at least three detection signals (210) using a given signature vector representing expected relative pulsatilities of the pulse signal in the at least three detection signals, wherein the computation of the pulse signal involves computing a weighted combination of the at least three detection signals using weights resulting in a pulse signal for which the product with the obtained at least three detection signals equals the relative pulsatilities as represented by the signature vector, and
   - a processing unit (133) for deriving physiological information (212) indicative of at least one vital sign from a part of said signature vector represented by vector coefficients that reflect pulsatilities of the detection signals comprising wavelength-dependent reflection or transmission information in wavelength channels above 600 nm.

2. Device as claimed in claim 1,
   wherein two of said at least three detection signals comprise wavelength-dependent reflection or transmission information in different wavelength channels of red and/or infrared light and the third detection signal comprises wavelength-dependent reflection or transmission information in a wavelength channel of visible light of another color than red.

3. Device as claimed in claim 2,
   wherein a first wavelength channel covers a wavelength or wavelength range in the range of 620 nm to 1000 nm, in particular in the range of 660 to 760 nm, a second wavelength channel covers a wavelength or wavelength range in the range of 620 nm to 1000 nm, in particular in the range of 850 to 940 nm, in particular around 900 nm, and a third wavelength channel covers a wavelength or wavelength range in the range of 400 nm to 600 nm, in particular in the range of 520 to 580 nm.

4. Device as claimed in claim 1,
   wherein said processing unit (132) is configured to derive physiological information (212) indicative of the relative concentration of a blood species, in particular of one or more of Hb, HbO2, MetHb, and HbCO.

5. Device as claimed in claim 1,
   wherein said pulse signal computation unit (133) is configured to compute at least two pulse signals (211a, 211b) from said at least three detection signals (210) using different given signature vectors, wherein for the computation of each pulse signal a different given signature vector is used, wherein a given signature vector represents expected relative pulsatilities of the respective pulse signal in the at least three detection signals, wherein the computation of a pulse signal involves computing a weighted combination of the at least three detection signals using weights resulting in a pulse signal for which the products with the obtained at least three detection signals equals the relative pulsatilities as represented by the respective signature vector, wherein said device further comprises a quality indicator computation unit (134) for computing quality indicator values for said pulse signals (211a, 211b) indicating a characteristic of the respective pulse signal, and wherein said processing unit (133) is configured to derive physiological information (212) indicative of at least one vital sign using a part of one or more signature vectors that result in the one or more pulse signals with the best quality indicator values and that is represented by vector coefficients that reflect pulsatilities of the detection signals comprising wavelength-dependent reflection or transmission information in wavelength channels above 600 nm.

6. Device as claimed in claim 5,
wherein said pulse signal computation unit (132) is configured to use a fixed set of different signature vectors and said processing unit (133) is configured to filter a time sequence of said part of the signature vectors that resulted in the pulse signal with the best quality indicator value to obtain a filtered partial signature vector from which the physiological information is derived, wherein said time sequence of partial signature vectors is obtained from pulse signals and quality indicators computed for successive time windows of said at least two detection signals (210).

7. Device as claimed in any one of claims 5 and 6,
wherein said quality indicator computation unit (134) is configured to compute the magnitude spectrum of normalized pulse signals, in particular over a sliding time window, and to use the amplitude of the highest peak in a range, in particular of typical pulse frequencies, as the quality indicator for said pulse signal.

8. Device as claimed in any one of claims 5 and 6,
wherein said quality indicator computation unit (134) is configured to compute the normalized magnitude spectrum of the pulse signal, and to use the amplitude of the highest peak in a range, in particular of typical pulse frequencies, as the quality indicator for said pulse signal.

9. Device as claimed in any one of claims 5 and 6,
wherein said quality indicator computation unit (134) is configured to compute pulse signals in a sliding time window and to define the skewness in the range of the pulse frequencies of the spectrum as the quality indicator for said pulse signal.

10. Device as claimed in any one of claims 5 and 6,
wherein said quality indicator computation unit (134) is configured to compute band-pass filtered pulse signals in a sliding time window and to define the kurtosis of a pulse signal as the quality indicator for said pulse signal.

11. Device as claimed in any one of the preceding claims,
wherein said pulse signal computation unit (132) is configured to compute said pulse signals $S_1$, $S_2$ by computing a covariance matrix $Q = C_n C_n^T$ of normalized DC-free detection signals $C_n$ over a time window and find the weights $W_x$ to compute a pulse signal $S_x = \overrightarrow{W_x} C_n$ as $\overrightarrow{W_x} = k\overrightarrow{P_{bvx}}Q^{-1}$, where k is chosen to make $\|\overrightarrow{W_x}\| = 1$ and $x \in \{1, 2\}$.

12. System for determining physiological information indicative of at least one vital sign of a subject, said system comprising:

    - a detector (112, 114) for detecting electromagnetic radiation transmitted through or reflected from a skin region of a subject and for deriving at least three detection signals ($C_n$) from the detected electromagnetic radiation, wherein each detection signal comprises wavelength-dependent reflection or transmission information in a different wavelength channel, wherein one of the detection signal comprises wavelength-dependent reflection or transmission information in a wavelength channel below 600 nm,
    - a device (130, 130a, 13b) as claimed in any one of the preceding claims for extracting physiological information.

13. Method for determining physiological information indicative of at least one vital sign of a subject, said method comprising:

    - obtaining at least three detection signals (210) derived from detected electromagnetic radiation transmitted through or reflected from a skin region of a subject, wherein each detection signal comprises wavelength-dependent reflection or transmission information in a different wavelength channel, wherein at least one of the detection signals comprises wavelength-dependent reflection or transmission information in a wavelength channel below 600 nm,
    - computing a pulse signal (211) from said at least three detection signals (210) using a given signature vector representing expected relative pulsatilities of the pulse signal in the at least three detection signals, wherein the computation of the pulse signal involves computing a weighted combination of the at least three detection signals using weights resulting in a pulse signal for which the product with the obtained at least three detection signals equals the relative pulsatilities as represented by the signature vector, and

- deriving physiological information (212) indicative of at least one vital sign from a part of said signature vector represented by vector coefficients that reflect pulsatilities of the detection signals comprising wavelength-dependent reflection or transmission information in wavelength channels above 600 nm.

14. Computer program comprising program code means for causing a computer to carry out the steps of the method as claimed in claim 13 when said computer program is carried out on the computer.

FIG.1

FIG.2

130a

210    131    210    132    211    133    212

FIG.3

130b

211a

210    131    210    132    211b    134    213    133    212

FIG.4

Index:

Set:

S100a    ▲ 1
S99a    .
S98a    .

1    .
.    .
.    .
.    .
S70a    ▼ 30

S100b    ▲ 31
S99b    .
S98b    .

2    .
.    .
.    .
.    .
S70b    ▼ 60
-
-      $SpO2 = 100 - mod(Index-1, 30)$
-
S100N    ▲ $1 + (N-1) \cdot 30$
S99N    .
S98N    .

N    .
.    .
.    .
.    .
S70N    ▼ $30 + (N-1) \cdot 30$

# FIG.5

Europäisches
Patentamt

European
Patent Office

Office européen
des brevets

## EUROPEAN SEARCH REPORT

Application Number

EP 19 15 8278

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/121834 A1 (KONINKLIJKE PHILIPS NV [NL]) 20 July 2017 (2017-07-20)<br>* abstract *<br>* page 1, line 2 - line 4 *<br>* page 3, line 29 - page 4, line 8 *<br>* page 5, line 28 - line 31 *<br>* page 7, line 7 - page 8, line 29 *<br>* page 10, line 18 - line 20 *<br>* page 12, line 28 - page 15, line 13 *<br>* page 17, line 3 - line 11 *<br>* page 17, line 21 - line 24 *<br>* page 18, line 10 - line 16 *<br>* page 19, line 30 - line 33 *<br>* page 20, line 16 - line 19 *<br>----- | 1-14 | INV.<br>A61B5/00<br>A61B5/0205<br>A61B5/024<br>A61B5/11<br>A61B5/1455 |
| X,D | WO 2017/055218 A1 (KONINKLIJKE PHILIPS NV [NL]) 6 April 2017 (2017-04-06)<br>* abstract *<br>* page 5, line 3 - line 21 *<br>* page 9, line 20 - line 25 *<br>* page 14, line 31 - page 18, line 8 *<br>* page 23, line 9 - line 21 *<br>* page 23, line 28 - page 24, line 2 *<br>* page 24, line 19 - line 23 *<br>* figures 1,2 *<br>-----<br>-/-- | 1-14 | TECHNICAL FIELDS SEARCHED (IPC)<br><br>A61B |

The present search report has been drawn up for all claims

| Place of search | Date of completion of the search | Examiner |
|---|---|---|
| The Hague | 24 June 2019 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

**EP 3 698 704 A1**

Europäisches
Patentamt

European
Patent Office

Office européen
des brevets

# EUROPEAN SEARCH REPORT

Application Number

EP 19 15 8278

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE HAAN G ET AL: "Improved motion robustness of remote-PPG by using the blood volume pulse signature", PHYSIOLOGICAL MEASUREMENT, INSTITUTE OF PHYSICS PUBLISHING, BRISTOL, GB, vol. 35, no. 9, 27 August 2014 (2014-08-27), pages 1913-1926, XP020269523, ISSN: 0967-3334, DOI: 10.1088/0967-3334/35/9/1913 [retrieved on 2014-08-27] * abstract * * page 1914, paragraph 3 * * page 1917, paragraph 3 * * page 1918 * * eq.(7), (8), (10) * * figures 2,7 * * sect.2.2 * | 1-14 | |
| A | Xueming Lin: "Using blood volume pulse vector to extract rPPG signal in infrared spectrum", , 1 January 2014 (2014-01-01), XP055598560, Retrieved from the Internet: URL:https://pure.tue.nl/ws/portalfiles/portal/47000435/785066-1.pdf [retrieved on 2019-06-21] * abstract * * sect. D * | 1-14 | TECHNICAL FIELDS SEARCHED (IPC) |

-/--

The present search report has been drawn up for all claims

| Place of search | Date of completion of the search | Examiner |
|---|---|---|
| The Hague | 24 June 2019 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

19

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | RICHARD MACWAN ET AL: "Remote photoplethysmography with constrained ICA using periodicity and chrominance constraints", BIOMEDICAL ENGINEERING ONLINE, vol. 17, no. 1, 9 February 2018 (2018-02-09), XP055598498, DOI: 10.1186/s12938-018-0450-3 * abstract * * page 8 - page 12 * * figures 1,4,5 * | 1-14 | |
| A | RICHARD MACWAN ET AL: "Heart rate estimation using remote photoplethysmography with multi-objective optimization", BIOMEDICAL SIGNAL PROCESSING AND CONTROL, vol. 49, 22 November 2018 (2018-11-22), pages 24-33, XP055598567, NL ISSN: 1746-8094, DOI: 10.1016/j.bspc.2018.10.012 * the whole document * | 1-14 | |

TECHNICAL FIELDS
SEARCHED       (IPC)

The present search report has been drawn up for all claims

| Place of search | Date of completion of the search | Examiner |
|---|---|---|
| The Hague | 24 June 2019 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 15 8278

This annex lists the patent family members relating to the patent documents cited in the above-mentioned European search report.
The members are as contained in the European Patent Office EDP file on
The European Patent Office is in no way liable for these particulars which are merely given for the purpose of information.

24-06-2019

| Patent document cited in search report | | Publication date | Patent family member(s) | | Publication date |
|---|---|---|---|---|---|
| WO 2017121834 | A1 | 20-07-2017 | CN | 108471989 A | 31-08-2018 |
| | | | EP | 3402402 A1 | 21-11-2018 |
| | | | JP | 2019505263 A | 28-02-2019 |
| | | | US | 2019000391 A1 | 03-01-2019 |
| | | | WO | 2017121834 A1 | 20-07-2017 |
| WO 2017055218 | A1 | 06-04-2017 | CN | 108366759 A | 03-08-2018 |
| | | | EP | 3355787 A1 | 08-08-2018 |
| | | | JP | 2018534968 A | 29-11-2018 |
| | | | US | 2017086755 A1 | 30-03-2017 |
| | | | WO | 2017055218 A1 | 06-04-2017 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013271591 A1

- WO 2017055218 A1